# EUROPEAN PATENT SPECIFICATION

(11) **EP 0 934 339 B1**
(45) Date of publication and mention of the grant of the patent: **03.09.2008**
(21) Application number: 97930416.9
(22) Date of filing: 26.06.1997
(51) Int. Cl.: C07K 16/44, C12P 21/00, A61K 39/395, G01N 33/94, G01N 33/543, C12N 9/96, A61K 39/385, C07K 16/06

(54) **Detoxification by intramuscular administration of F(ab') 2 fragments**
Detoxifizierung durch intramuskulär verabreichte F(ab')2 Fragmente
Détoxification par l'administration intramusculaire de fragments F(ab')2

(30) Priority: 27.06.1996 IT MI961309
(43) Date of publication of application: 11.08.1999
(73) Proprietor: DOX-AL ITALIA S.p.A., I-20050 Correzzana Milano (IT)
(72) Inventor: VOLPATO, Ivo, I-06070 S. Mariano (IT); BIZZINI, Bernard, F-46100 Figeac (FR); GRABITZ, Ernst, Bernhard, I-22064 Campofiorenzo Fraz. di Casatenov (IT)
(74) Representative: Gervasi, Gemma
(86) International application number: PCT/EP1997/003358
(87) International publication number: WO 1997/049732

(56) References cited:
- FR-A- 2 301 266
- K. AOKI ET AL.: "Immunoassay for methamphetamine with a new antibody." FORENSIC SCIENCE INTERNATIONAL, vol. 44, no. 2-3, February 1990, IRELAND, pages 245-255, XP000673787
- B. ROSE ET AL.: "Competitive indirect ELISA for ceftiofur sodium and the effect of different immunizing and coating antigen conjugates." BIOCONJUGATE CHEMISTRY, vol. 6, no. 5, September 1995, WASHINGTON, DC, USA, pages 529-535, XP002031389
- M. HURSTING ET AL.: "Tricyclic antidepressant-specific Fab fragments alter the distribution and elimination of desipramine in the rabbit: A model for overdose treatment." JOURNAL OF TOXICOLOGY. CLINICAL TOXICOLOGY, vol. 27, no. 1-2, 1989, NEW YORK, NY, USA, pages 53-66, XP000673781
- D. MONGKOLSIRICHAIKUL ET AL.: "Development of a latex agglutination inhibition reaction test for amphetamines in urine." JOURNAL OF IMMUNOLOGICAL METHODS, vol. 157, no. 1-2, 4 January 1993, AMSTERDAM, NL, pages 189-195, XP002031390
- REMCO DE BREE et al., Clinical Cancer Research (March 1995), Vol. 1, pages 277-286
- TIBBEN J. et al., International Journal of Cancer, (May 1996), Vol. 66, No. 4, pages 477-483
- BRUNN GJ. et al., Int. J. Immunopharmac. (1991), Vol. 13, No. 7, pages 841-851
- BOSSE GM. et al., The Journal of Emergency Medicine (1994), Vol. 12, No. 2, pages 179-185
- ALBERT H *a STAINES N. (EDITORS): *dMethods of Immunological Analysis*d, 1993, VCH VERLAGSGESELLSCHAFT, WEINHEIM, GERMANY
- PEPIN S; ET AL: 'SNAKE F(AB'S)2 ANTIVENOM FROM HYPERIMMUNIZED HORSE: PHARMACOKINETICS FOLLOWING INTRAVENOUS AND INTRAMUSCULAR ADMINISTRATIONS IN RABBITS' PHARMACEUTICAL RESEARCH vol. 12, no. 10, October 1995, NEW YORK, NY, US, pages 1470 - 1473, XP001085374

## Description

### 1. Field of the invention

The present invention relates to the use of F(ab')₂ fragments active as specific antibodies to drugs and metabolites thereof, in the detoxification of humans and animals from said drugs.

Detoxification (i.e. removal of exogenous substances from organism) is extremely useful to neutralize the toxic effects that are induced. e.g., by drug overdose, accumulation, poisoning and, in man, also by drugs abuse.

It is of particular importance in the zootechnical field because, in the latest years, the search for ever new production goals and the growing development of intensive commercial-scale breeding spurred the recourse to drugs, whereby the traditional production rhythms are deeply affected.

Several medicinal substances are used in zootechny, e.g., (i) to prevent and treat infectious diseases, in particular the zoonotic ones, i.e., diseases or infections common to animals and humans and mutually communicable, representing about half of the over four hundred infections or infestive diseases, which are a prospective risk for livestock; (ii) to prevent infestations; (iii) to improve the growth rate and increase food utilization; (iv) to promote milk production.

Out of the drugs used in the zootechnic field to enhance livestock performance, i.e., increase the assimilation rate and favour a more complete food utilization, the auxinic, anabolic drugs, anti-thyroid agents, cortisone, corticosteroids and their synthetic analogs, and sympathicomimetic drugs are the most widely used.

Out of the auxinic drugs, mention is made of virginiamycin, zincobacitracin, avoparicin antibiotics, which act on the microflora of the alimentary canal and improve food assimilation.

Out of the anabolic drugs, mention is made of native endocrine hormones, the most widely used in zootechny being 17-β-estradiol, testosterone, progesterone, exocrine hormones obtained by synthesis, the most widely used in zootechny being 19-nortestosterone (nandrolone), and trenbolone, which are androgenic, and zeranol and diethylstilbestrol (DES) which are estrogenic.

Among anti-thyroid treating agents, the most widely used are methylthiouracil and propylthiouracil.

Cortisone and related drugs endowed with corticosteroid activity, hereinafter referred to as corticosteroid analogs, act on the glucidic and protidic metabolism and cause retention of fluid; they can be combined with endogenous hormones as low-absorption dexamethazone based compositions.

The most widely used sympathicomimetic drugs are β-agonist clenbuterol and cimaterol, which favour lipolysis and proteic synthesis.

Finally, somatotropin is used to enhance growth and milk production.

The advantages obtained in terms of simplicity of use and results achieved are undeniable. However, there are a series of risks for a consumer, due to the presence of medicaments or of their active metabolites in the organism or in milk and meat derived from treated animals. Therefore, a sufficient time for total drug elimination from the organism has to elapse before animal slaughtering or milk collection to safeguard consumers from the potential risk of the side effects thereof, which is particularly high in low-bodyweight subjects, such as sucklings and babies.

The lack of rapid and accurate diagnostic methods useful to detect circulating levels of drugs and metabolites thereof to be performed directly at farms further complicates the possibility of an *ad hoc* therapeutic intervention and, in some cases, favours a great number of drugs or higher dosage rates to be administered.

### 2. Technical problem

It follows that there is an urgent need for methods of control of the presence of circulating medicinal or toxic substances as well as for an as rapid as possible method of their elimination, not involving further risks for the subject treated and, in case of livestocks, for the consumer of zootechical products derived therefrom.

### 3. State of the art

The immunoglobulino-therapy (serotherapy) of infectious diseases is known in clinical practice: gamma-globulins are administered (passive immunization), optionally in combination with active immunization.

### 4. Summary

The Applicant has surprisingly found that drugs and/metabolites thereof can be more rapidly eliminated from human and animal bodies by administering F(ab')₂ fragments of IgG immunogobulins, active as specific antibodies to said medicaments or drugs and metabolites.

According to the present disclosure drugs or metabolites, (haptens) which are unable by themselves to stimulate an immune response from the body, are converted, by conjugation with heterologous proteins or polysaccharides, into immunogen derivatives, i.e., capable of stimulating the formation in the treated animals of IgG immunoglobulins active as specific antibodies to said drugs or metabolites.

IgG immunoglobulins obtained from animal serum are used to prepare F(ab')₂ fragments by removal of the Fc fragment; they may also be used as are for detoxifying treatments and as diagnostic reagents in a method of qualitative or quantitative immunodiagnostic determinations of the concentration in a biological fluid, of a drug and/or metabolites thereof, whereto they act as specific antibodies.

The present disclosure relates to the aforesaid F(ab')₂ fragments and

IgG immunoglobulins, the pharmaceutical compositions containing same, the method of their preparation, and their use for the detoxification of humans and animals.

The disclosure also includes the aforesaid immunogen derivatives and the preparation process thereof; the aforesaid immunodiagnostic method and the corresponding diagnostic kit; drug-enzyme conjugates useful as diagnostic reagents for the aforesaid diagnostic method and the method of their preparation, as disclosed in the present text.

The F(ab')₂ fragments enhance the in vivo elimination of drugs and/or metabolites from the body, presumably by forming antigen-antibody complexes which promote macrophages opsonization and phagocitosis, with subsequent increase in elimination.

### 5. Detailed description of the invention

The molecular weight of the claimed IgG immunoglobulins is of about 150.000 Daltons; that of the corresponding F(ab')₂ fragments is lower [e.g. of about 100,000 daltons].

The claimed F(ab')₂ fragments find extensive application in the detoxifying treatment of drug-treated humans and animals, e.g., in case of drug poisoning, overdose, for instance in humans in case of abuse of pharmacologically active substances.

The claimed F(ab')₂ fragments find in particular application in the zootechnical field in the detoxification of drug-treated livestock (e.g. mammals such as cattle, sheep, pigs) before slaughtering or milk collection.

The removal of Fc fragments from the present IgG immunoglobulins allows them to be despeciated and to increase organism's tolerance towards them. In any case, the detoxifying treatment is well tolerated also when the present IgG immunoglobulins are administered to animals belonging to the same species as that utilized for their production by immunization.

The present detoxifying treatment is, e.g., meant for promoting the elimination of the following drugs from the organism:
- antibiotics, e.g., tetracyclines (such as oxytetracycline, doxycycline); amino glycosidic antibiotics (such as neomicyn B, gentamicin C. tobramycin, kanamycin, amikacin, and streptomycin; cephalosporins (e.g. ceftazidime, cefotaxime, cephalexin); penicillins;
- sulphonamides (derived from sulphanilamide, e.g., substituted with an isoxazole ring at the sulphonamide nitrogen, such as sulphamethoxazole and sulphamethazine;
- antiviral drugs, such as idoxuridine;
- antiparasitic drugs;
- antimycotic drugs (amphotericin, ketoconazole);
- antitubercular drugs, such as cycloserine, isoniazid;
- sympathicomimetic drugs, in particular having 2-phenylethylamine structure, e.g., β-agonist such as clenbuterol and cimaterol;
- anti-thyroid treating agents, in particular 2-thiouracils (e.g. methylthiouracil and propylthiouracil);
- estrogenic substances, used, e.g., as anabolic steroids in zootechny, such as diethylstilbestrol, 17-β-estradiol, zeranol;
- androgenic substances, used, e.g., as anabolic steroids in zootechny, such as trenbolone, 19-nortestosterone (nandrolone);
- progestinic substances (progesterone);
- cortisone and corticosteroid analogs, e.g. adrenocorticosteroids, such as dexamethasone;
- other endogenous hormones, such as somatotrophin (growth hormone STH);
- auxinic drugs, out of which antibiotics, such as virginiamycin, zincobacitracin, avoparcin;
- amphetaminic drugs (amphetamine, methamphetamine);
- growth stimulants, e.g. with quinoline structure such as olaquindox;
- disinfectants.

According to the present invention, the claimed antibody F(ab')₂ fragments (or the claimed IgGs) are administered to the subject to be detoxified by the intramuscular (i.m.) route. Preferably, prior to their administration, the hematic, serum or milk concentration of the drug to be eliminated is determined to ascertain more accurately the dose of immunodetoxifier to be administered.

To obtain a substantial drug elimination within a short time (e.g. within 24 to 48 h). F(ab')₂ are preferably administered at a dose corresponding to F(ab')₂: drug or metabolite molar ratios for instance comprised between 1:1 to 1:5. referred to drug or of metabolite present in the blood of the subject to be treated [each mol of F(ab')₂ fragment or of IgG can bind a molar amount of drug equal to or higher than 1 mol of drug].

When integral IgG are used, they can be administered at a dose of 1 mole of immunoglobin per 10 or more than 10 mole of drug or metabolite present in circulating blood, depending on the antibody's (immunoglobin's) avidity.

Should drugs and/or metabolites be still present in the blood, said first F(ab')₂ (or IgG) administration will be followed, if so required, by one or more further administrations.

In particular, the determination of the drug and/or metabolite concentration in the blood of the subject to be treated is to be repeated about 24 to 72 h after each administration of the claimed F(ab')₂ fragments (or IgG) to check whether some drug and/or metabolites thereof are still present in the blood. F(ab')₂ fragments (or IgGs) are typically administered in the form of pharmaceutical compositions including at least one of the aforesaid fragments (or IgGs), in a therapeutically effective amount, combined with one or more pharmaceutically acceptable excipients or diluents, either singly or as a mixture thereof, and may. e.g., be prepared by conventional methods, like those reported in Remington's Pharmaceutical Sciences, 18th Ed., 1990. Particularly preferred are the preparations for injection, comprising at least one of the aforesaid fragments (or IgGs), at least a diluent (e.g., a solvent, such as physiological saline solution, wherein they are dispersed, dissolved or emulsified), and other excipients, if any. The preparations for injection may be in the form, e.g., of two vials, one enclosing the F(ab')₂ fragments (or IgGs) in the solid state and the other enclosing the diluent or solvent.

Pharmaceutical composition can be also in the retard form.

Preparation of F(ab')₂ fragments: they are obtained by removing by digestion with pepsin the Fc fragment of the corresponding IgG immunoglobulins obtained from the serum of animals treated with immunogen derivatives, these last being obtained by conjugating the compound selected as the hapten for the drug to be removed from the body with a proteic or polysaccharide carrier.

Preparation of immunogen derivatives

The carrier used for the preparation of immunogens is typically a heterologous protein i.e. derived from an animal of a species differing from that used for the production of IgG antibodies, or a polysaccharide. Exemplary proteins that may be used as carriers are bovine serum albumin (BSA), human serum albumin (HSA), chicken ovoalbumin (OVA), bovine globulins, chicken or turkey globulins, limset hemocyanin. Bovine or human serum albumins, chicken ovoalbumin and gamma-globulins are preferably used to produce antibodies in animals, such as rabbits, goats or horses.

Hapten: proteic carrier molar ratios found in the immunogens or used for immunogen preparation depend on the proteic carrier molecular weight.

Hapten: proteic carrier molar ratios founds in the immunogens range for instance about from 15:1 to 50:1, e.g. about from 15:1 to 20:1 in the case of HSA or BSA, and in general of proteic susbtances with molecular weight of about 40,000-70,000 daltons (included those used for conjugation for diagnostic purposes), and are about proportionally higher in the case of higher molecular weight proteins, e.g. up to 2-3 folds higher in the case of IgGs having M.W. 160,000 daltons.

Unless otherwise reported, hapten is typically added to the proteic substance both for the preparation of immunogens or of diagnostic conjugates) in further excess with respect to that found in the final product; the hapten: proteic carrier molar ratios added to the reaction mixture range for instance about from 100:1 to 200:1 for HSA or BSA, which has a molecular weight of about 65,000 daltons; e.g. from about 20:1 to 60:1 for OVA.

The unreacted hapten excess is suitably removed from the reaction mixture, e.g. by dialysis.

Proteic or polysaccharidic carriers have for instance molecular weights ranging from 40,000 to 450,000 Daltons (for example, BSA having a MW of 65,000).

The compound conjugated with the carrier to produce an immunogen of said compound is called hapten. The hapten preferably utilized is the substance to be eliminated from the organism, and can be the same drug administered to the subject to be treated, a metabolite thereof, if said metabolite is the active compound to be eliminated from the organism, or a structurally related derivative thereof, e.g., a derivative thereof, functionalized with functional groups suitable for the covalent conjugation with the proteic or polysaccharide carrier to be obtained.

For example, the hapten preferably used for cephalosporins is the same cephalosporin administered to the subject to be detoxified. Should the drug to be eliminated be a penicillin, the hapten preferably used will be the metabolites thereof, 6-aminopenicillanic acid (6-APA) or penicilloic acid.

The hapten preferably used for tetracyclines is the same drug administered, preferably functionalized as illustrated in the present text.

Examples of tetracyclines used as drugs and as haptens are chlortetracycline, oxytetracycline, demethyltetracycline, methacycline, doxycycline, and minocycline.

The hapten preferably used for amino glycosidic antibiotics is preferably the specific antibiotic administered to the subject to be detoxified.

The hapten preferably used for symphaticomimetic drugs having 2-phenylethylamine structure is the same drug administered to the subject to be detoxified.

As concerns cephalosporins, penicillins, tetracyclines and sympathicomimetic drugs having 2-phenylethylamine structure and particular examples thereof, reference is made to Goodman & Gillman, "The pharmacological basis of therapeutics", The Macmillian Co., 3rd Ed., 1966, pp. 1194-1227, in particular p. 1195, pp. 1242-1257, p. 484. and to the translation into Italian of the 6th American edition of Goodman and Gilman's book: "Le basi formacologiche della terapia", Editoriale Grasso, Bologna, 1982, pp. 1234-1239 and p. 1265.

The hapten typically used for sulphonylamides is the same compound administered to the subject to be detoxified, or a sulphonamide substituted at the -SO₂NH₂ sulphonamide nitrogen with an aromatic ring of the same type as that present in the drug. For example, for sulphamethoxazole, sulphamethoxazole itself or another sulphonamide containing an optionally substituted isoxazole ring is used.

The hapten preferably used for anti-thyroid 2-thiouracyl derivatives is a 2-thiouracyl derivative substituted at position 6 with a C₁-C₃ alkyl, preferably functionalized as illustrated in the present text.

The hapten preferably used for diethylstilbestrol, zeranol or trenbolone is the same drug administered, preferably functionalized as hereinafter illustrated. The hapten preferably used for 17-β-estradiol and derivatives thereof (e.g. esters, such as estradiol 17-β-cypionate and estradiol 3,17-β-dicypionate) is 17-β-estradiol, preferably derivatized as hereinafter described.

The type of carrier and hapten-carrier conjugation depends on the chemical structure of the drug and/or metabolite to be converted into immunogen. Conjugation generally takes place by formation of one or more covalent bonds between the hapten and the carrier.

Some methods of preparation of immunogens are reported below by way of illustration.

### Immunogen derivatives represented by hapten/proteic carrier conjugates obtained by treating a hapten with a proteic carrier in the presence of a carbodiimide

The method is suitable for the conversion into immunogens of haptens containing -NH₂ aliphatic or aromatic primary amino groups, or -COOH carboxy groups.

The -COOH groups of the proteic carrier (or hapten), activated by carbodiimide reaction through formation of the corresponding O-acylisourea, react with the -NH₂ groups of the hapten (or proteic carrier) with formation of an amide bond.

The proteic carrier can be e.g. BSA, HSA, OVA or IgG, for instance HSA or OVA for method A, and gamma-globulin IgG for method B, as hereinafter illustrated; the carbodiimide is preferably N-ethyl-N'-(3-dimethylaminopropyl)-carbodiimide or another pharmaceutically acceptable carbodiimide, giving pharmaceutically acceptable reaction products (e.g., dialkylcarbodiimides containing C₁-C₁₂ alkyl groups, optionally substituted with a tertiary amino group, such as -N(CH₃)₂).

### Method A - (Carbodiimide direct conjugation method) - Immunogen derivatives obtained by conjugation of a hapten directly with a proteic carrier, in the presence of a carbodiimide, for instance by preactivation with carbodiimide of the compound containing the -COOH groups.

The hapten/proteic carrier conjugate is obtained, e.g., by causing the compound containing -COOH groups (e.g., the proteic carrier, in case of haptens containing NH₂ groups or a hapten containing -COOH groups) to react with A carbodiimide in a stoichiometric excess with respect to the -COOH groups, typically in an aqueous medium, generally at an alkaline pH (e.g., 8-10), at about 10°C to 40C (e.g., 37°C). The resulting product is treated with a compound containing -NH₂ groups (the hapten containing -NH₂ groups, or the proteic carrier, if a hapten containing -COOH groups has been activated with carbodiimide).

According to some specific embodiments hapten, proteic carrier and carbodiimide, e.g. N-ethyl-N'-(3-dimethylaminopropyl)-carbodiimide, are for instance reacted in aqueous medium, e.g. at pH 5.0-6.0. e.g. 6.0, with excess carbodiimide, at a temperature e.g. of +30°C, e.g. at +10°C.

Hapten: proteic carrier molar ratios reacted for the preparation of immunogen can be for instance of from 100:1 to 200:1 for BSA, HSA and from 20:1 to 60:1 for OVA.

In any case, carbodiimide condensation may be carried out according to the method disclosed by E.C. Beuvery, G.J.A. Speijers, B.I.G. Lutz, D. Freudenthal, V. Kanhai, B. Haagmans, and H.J.G.M. Derks, in Develop. Biol. Standard, S. Karger, Basel (1986), 63, 117.

### Method B - Immunogen derivatives obtained by conjugation, in the presence of a carbodiimide, of a hapten containing primary -NH₂ groups, with a proteic or polysaccharidic carrier previously oxidized and conjugated with a poly-amino acid copolymer.

According to a variant developed by the Applicant, the hapten is conjugated with a proteic or polysaccharidic carrier, e.g. with a glycoproteic carrier, through a bridge of a poly-amino acid copolymer, such as Poly.(D-Glu, D-Lys). The relevant procedure, described hereinafter in greater detail with reference to a proteic carrier substance, comprises the step of:
a) treating a proteic substance with an oxidizing agent of the -C-OH groups to C=0 groups;
b) treating the resulting oxidized proteic substance with a synthetic poly-amino acidic copolymer, and the product thus obtained with a reducing agent, such as NaBH₄;
c) treating the resulting oxidized proteic substance/amino acid copolymer conjugate with the hapten, in the presence of a carbodiimide.

In the present text, the -C-OH groups are alcoholic groups and the oxidizing agent is more particularly an oxidizing agent of primary alcoholic groups to aldehydic -CHO groups.

An advantage offered by this method is the possibility of controlling accurately the amount of hapten that links to the proteic carrier (which is still found in the final product in an amount substantially corresponding to the amount caused to react).

In immunogens preparation, the proteic substance is the carrier and the hapten is the drug or the metabolite thereof to be detoxified, or the derivatives thereof selected as a hapten for said drug.

According to method B, the proteic carrier can have molecular weight 40,000-70,000, such as BSA, HSA with molecular weight 65,000, or can be a glycoprotein, for instance a gamma-globulin IgG (for instance with a molecular weight of 160,000 Daltons). Alternatively, the carrier can be a polysaccharidic polymer with molecular weight comprised between about 50,000 and 200,000 Daltons, such as for instance dextrans or alginates.

Oxidation of the proteic substance is typically carried out with periodate, typically an inorganic periodate, e.g. in water, at a pH preferably ranging from 4.5 to 7.5. preferably at +4°C to 40°C, and shielded from light.

Oxidation is typically carried out with NaIO₄. at a pH of 5.0 (e.g., in acetate buffer), at room temperature (about 20°C to 25°C).

The oxidizing agent is typically in a stoichiometric excess with respect to the -OH groups of the proteic substance, preferably of 0.01M-0.1 moles of NaIO₄ each 10⁻⁷ - 10⁻⁸ moles of proteic substance, e.g. of glycoproteic carrier.

The reaction is generally complete after about 15-60 min and the excess of oxidizing agent is generally removed by addition of molar excess of alcohol, typically glycerol or ethylene glycol, at the abovementioned oxidation temperature. Before performing the successive step, the reaction mixture is generally allowed to stand at 0°C/+5°C to allow excess NaIO₄, to react with the alcohol.

The poly-amino acid copolymer is typically a copolymer containing acid α-amino acids (which contain at least a further -COOH group or another acidic group in addition to the α-carboxy group) and basic α-amino acid (which contain other -NH₂ groups or other basic groups in addition to the α-amino group). The copolymer has a molecular weight for instance of 20,000-100,000 daltons, preferably 50,000, and contains L or D amino acidic residues, or mixtures thereof. It contains different aminoacidic sequences, in different ratios, provided that it contains glutamic acid /Glu) and Lysin (Lys).

Preferably, a D-Glu,D-Lys copolymer having a MW typically ranging from about 20,000 to 100,000, preferably from 20,000 to 50,000 is used, such as the Poly(D-Glu, D-Lys) copolymer having a MW of about 50,000 and a Glu:Lys ratio equal to 6:4, available under the trademark Sigma ^{R} P7658.

Condensation b) between the oxidized proteic substance and the copolymer is generally carried out in an aqueous medium, at an alkaline pH, e.g., of about 8-10, generally at a temperature ranging from 4°C to 40°C, e.g. at 4°C to 8°C overnight (e.g., in a refrigerator), typically from 30°C to 40°C for about 2 to 4 h, and shielded from light. Typically, the condensation is carried out at a pH of about 9 (e.g. in CO₃⁻⁻/HCO₃ buffer), at 37°C., for 2-4 hours.

The oxidized protein/copolymer molar ratio used in the reaction medium typically ranges from 1:5 to 1:30, e.g., 1:15, which is substantially found in the conjugate obtained.

The aforementioned reducing agent of step b) is typically NaBH₄, typically used in molar excess, e.g. at about 0°C to 10°C, generally at 4°C to 6°C for 2-6 hours, typically 4 hours.

This treatment with reducing agent has the scope of reducing the double bond (HC=N-) formed during condensation according to step b), allowing stabilization of the final compound.

The oxidized proteic substance/poly-amino acid copolymer conjugate so obtained is typically purified by dialysis on membranes with molecular exclusion limit (cutoff) typically ranging from 200,000 to 300,000, e.g. about 200,000 Daltons (200 KD), against a buffer at a pH of about 5.0 to 6.0, e.g.. 5.5, and preferably at cool temperature, at about 0°C to 10°C, generally at 4°C to 8°C. Separation from non-oxidized protein residue is also obtained.

The condensation between the hapten and oxidized carrier, conjugated with the copolymer [phase c)], is typically carried out with N-ethyl-N'-(3-dimethylaminopropyl)-carbodiimide.

The condensation is typically carried out in an aqueous medium, at a pH preferably ranging from 5.0 to 6.0 (e.g.. 5.5, in acetate buffer). e.g. at about 30°C to 40°C, generally at 37°C. For instance, aqueous solutions of hapten and proteic substance/poly-amino acidic copolymer conjugate are mixed (e.g., in the concentrations reported in the Experimental Part), carbodiimide is added and the reaction temperature is brought to the selected value.

The hapten added to the proteic substance substantially corresponds to the molar ratio hapten: proteic substance found in the final conjugate, which is preferably of about 15:1 to 50:1, typically 15:1 in respect of the poly-amino acidic copolymer present in the proteic substance/poly-amino acidic copolymer conjugate.

The amount of copolymer substantially corresponds to the amount added to the oxidized proteic substance, as proved by proteic content determination and electrophoretic analysis carried out according to conventional techniques.

Carbodiimide is typically used in a molar excess with respect to the - COOH groups of the copolymer-protein conjugate, e.g., of about 3 - 6 mmol per 1-10 µmol of protein/amino acidic copolymer conjugate, these molar ratios being in particular referred to a conjugate between a glycoprotein carrier with a M.W. in the range 65,000-200,000 daltons and a poly-amino acid copolymer with a M.W. of about 50,000 daltons.

The hapten/carrier conjugate is typically purified by dialysis, by causing it to pass through membranes with molecular exclusion limit (cutoff) value of about 10,000 to 300,000 Daltons, depending on molecular weight of the copolymer and of the protein, preferably of about 12,000 Daltons, against a buffer at a pH of about 6.0 to 8.0, preferably of 7.0 to 7.5, e.g., against PBS (physiological saline solution/phosphate buffer), operating preferably at 4°C to 10°C (e.g., about 4°C). Low-molecular-weight fragments and salts are thus eliminated.

Conjugation of haptens with carriers in the presence of carbodiimide applies to haptens with primary amino groups -NH₂ or with -COOH groups, direct conjugation according to method A) being for instance applied to haptens with -COOH or -NH₂ groups, and conjugation via poly-aminoacid copolymer bridge (i.e. method B) being for instance applied to haptens with primary amino groups -NH₂.

The immunogen is the conjugation product through one or more covalent bonds between the hapten and the proteic carrier, either as such or as oxidized glycoproteic carrier/poly-amino acido copolymer conjugated, for instance:
- amide bonds between -COOH groups of haptens and -NH₂ groups of proteic carriers, according to the direct method proteic carrier-haptens, as for instance for cephalosporins and penicillins metabolites;
- amide bonds between -NH₂ groups of haptens and -COOH groups of the glycoproteic carrier, according to the method carrier-copolymer-hapten (Method B.), as for instance for aminoglycosides, cycloserine, amphetamine, sulphamethoxazole.

Conjugation in the presence of carbodiimide either with a proteic carrier as such (Method A), or with a glycoprotein oxidized and conjugated with an poly-amino acidic copolymer as described above as to Method B. is applied, e.g., to the conversion into immunogens of the following compounds:
Method A [protein-hapten *via* carbodiimide direct conjugation method; haptens react with their -COOH groups] is in particular suitable for:
   - cephalosporins, for instance ceftazidime, cefotaxime, cephalexin (cephalosporins typically react with the -COOH at position 4);
   - 6-amino penicillanic acid and penicilloic acid, selected as haptens for the preparation of F(ab')₂ active as antibodies to penicillins.
Method B [conjugation protein-hapten-copolymer, preferably glycoprotein-hapten-copolymer] is in particular suitable for conversion into immunogens of the following compounds containing primary aliphatic or aromatic groups:
   - amino glycosidic antibiotics, e.g. neomycin, gentamicin, streptomycin;
   - antimycotic drugs, e.g. amphotericin;
   - antitubercolar drugs, e.g. cycloserine;
   - sulphonylamides, e.g. sulphametoxazole;
   - amphetamine;
   - clenbuterol, cimaterol.
Drugs or metabolites can be functionalized so to allow at least one - COOH group to be introduced in their molecule. For instance, drugs or metabolites containing aromatic groups activated towards diazotization (i.e. towards electrophilic substitution with diazonium reagents) e.g. containing one or more phenolic groups, such as
   - tetracyclines, such as oxytetracycline;
   - steroids or analogs thereof containing at least one phenolic group - OH, e.g. 17-beta-estradiol, diethylstilbestrol, zeranol,
   - or compounds with a quinoxaline ring, such as olaquindox,
   can be converted into reactive haptens, by reacting them with diazotized para-amino benzoic acid, which affords the corresponding 4-carboxyphenyl azo derivative, thus allowing a -COOH group to be introduced in the drug molecule (for instance according to the procedure of N. Weliky, H.H. Weetall, Immunochemistry, 2, 293, 1965), then conjugated with a carrier, for instance with BSA, HSA or OVA in the presence of a carbodiimide, according to Method A.

Examples are the conjugation product of HSA with 5-azo-(4-carboxyphenyl)-N-(2-hydroxyethyl)-3-methyl-2-quinoxaline carboxyamide (i.e. the reaction product of olaquindox with diazotized para-amino benzoic acid), and the conjugation product between HSA and the reaction product of oxytetracycline with diazotized para-amino benzoic acid, both obtained in the presence of carbodiimide, such as the conjugates according to examples 9d and 10d, respectively.

Drugs or metabolites with keto groups can be reacted with compounds containing both a carboxylic group and a function able to react via nucleophilic attack with the keto group (such as hydroxylamines or hydrazines carrying also carboxy groups, either as such or masked), thus allowing a carboxy group to be introduced into the molecule, then conjugated with a carrier, e.g. with BSA, according to Method A.

For instance, compounds with keto groups can be reacted with 0-(carboxymethyl)-hydroxylamine [i.e. carboxymethoxylamine or aminoxy-acetic acid], following e.g. to the procedure of R. J. Warren and Fotherby, J. Endocrinol., 1974, 62, 605. thus allowing a carboxy group to be introduced into the hapten molecule, and the corresponding 0-(carboxymethyl)oxyme derivatives thus obtained are then conjugated with a carrier, e.g. with BSA, according to Method A: for instance, ketokonazole was converted into immunogen by this route.

Compounds with keto groups (e.g. trenbolone) can be for instance converted into the corresponding hydrazones containing a -COOH group by treatment with para-hydrazinobenzoic acid, following for instance the procedure of B. Africa. E. Haber, Immunochemistry, 8, 479, 1971, and the derivatives thus obtained are conjugated with a carrier, e.g. with BSA, according to Method A.

Secondary amines, for instance aliphatic ones such as methamphetamine, can be for instance converted into reactive haptens by reaction with N-(4-bromobutil)phtalymide, e.g. following the procedure of G.W. Aherne, E.M.Piall, V. Marks, in Br. J. Clin. Pharmacol., 3, 561, 1976, then hydrolizing the phtalymido group to give the corresponding primary amines, i.e. the corresponding primary amine with the 4-aminobutil residue linked to the hapten secondary nitrogen, which are then reacted with the -COOH groups of a proteic carrier, e.g. with BSA, according to Method A.

### Method C - Immunogen derivative obtained by conjugation of a proteic carrier with a hapten having -C-OH- groups oxidized to C=O groups, followed by reduction with NaBH₄.

Amino glycosidic antibiotics and other drugs containing -C-OH groups in a glucydic mojety, such as neomycin, gentamicin, streptomycin, the antimycotic drug amphotericin, and idoxuridine are suitably converted into immunogens by treatment with an oxidizing agent capable of oxidizing the -C-OH groups to C=O groups, followed by treatment with a proteic carrier, e.g. BSA, HSA then by treatment with NaBH₄.

Hapten oxidation is typically carried out with excess periodate, such as NaIO₄, in an aqueous medium, at a pH preferably ranging from 5.0 to 7.5, e.g., at a pH of 5.5 (e.g., in acetate buffer), at temperatures generally ranging from 4°C to 40°C preferably at room temperature (+20°C/+25°C) or at 37°C, and shielded from light. The excess of oxidizing agent is typically neutralized by glycerol or ethylene glycol addition, as described above for the oxidation of proteic carriers; the reaction of oxidized hapten and protein (typically BSA or HSA having a MW of 65.000) is typically carried out at an alkaline pH (preferably of about 8.5 to 9.5) at the oxidation temperature specified above.

### Method D - Immunogen derivative obtained by conjugation of a hapten with a proteic carrier with -C-OH groups oxidized to C=O groups followed by reduction with NaBH₄

The method concerns the preparation of hapten/proteic carrier conjugates, of haptens containing primary -NH₂ groups, e.g., amino glycosidic antibiotics, amphotericin, and proteic carriers oxidized with oxidizing agents capable of oxidizing the -OH groups to C=O groups.

The proteic substance (a carrier in the case of immunogens) is typically oxidized with periodate, under the aforementioned conditions (step a). The excess oxidizing agent is eliminated by glycerol addition as described above. The successive conjugation with the hapten is typically carried out at an alkaline pH (e.g., of about 8.5 to 9.5), at temperatures generally ranging from 4°C to 40°C (e.g., at 4°C to 8°C overnight or at 20°C to 37°C for about 2 to 6 h) and is followed by treatment with NaBH₄. The proteic carrier can be for instance BSA (e.g. having a MW of 65,000), or HSA and is oxidized with periodate; the drugs converted into immunogens are, e.g., neomycin, gentamicin, streptomycin, and amphotericin.

### Method E - Immunogens obtained by conjugation of the hapten with a proteic carrier in the presence of glutaraldehyde

The method is suitable for the conjugation of haptens containing primary aliphatic or aromatic -NH₂ amino groups with proteic substances.

Under typical conditions, glutaraldehyde is added to a mixture of proteic substance and hapten, in an aqueous medium, at a pH generally ranging from 6.0 to 8.0, preferably from 6.7 to 6.9 (e.g., in phosphate buffer), at about 4°C to 40°C, typically at room temperature (20°C to 25°C).

The reaction is complete in about 0.1 to 5h, typically in 3 hours.

Glutaraldehyde is typically used in stoichiometric excess with respect to the -NH₂ groups of the proteic carrier, for instance in amounts of 10⁻⁴ to 10⁻⁵ moles each 10⁻⁵/10⁻⁶ moles of -NH₂ groups of the proteic substance (e.g. BSA has about 59 moles of -NH₂ groups per mole of BSA). For instance, glutharaldehyde is used in an amount ranging from 0.1 to 1.0 mg, e.g., 0.5 mg per mg of proteic substance. Once the reaction is complete, excess glutaraldehyde is neutralized by treatment with lysine which is typically used in an amount of about 1.5 to 15 mg, e.g., 7.5 mg per mg of proteic carrier), under the same operating conditions used for the conjugation with the hapten.

The hapten/proteic substance molar ratio preferably ranges from 10:1 to 50:1, and more preferably is equal to 20:1.

For immunogens preparation, the proteic substance is for instance BSA (e.g., having a MW of about 65,000 Daltons) or HSA.

The resulting hapten/proteic substance conjugates, and more generally all hapten/proteic substance conjugates described herein, are preferably purified by dialysis, typically through membranes with molecular exclusion limit (cut-off) of about 10,000 daltons (10 KD), preferably against a buffer with pH 6.0-8.0, for instance pH 7.0-7.5. such as PBS (saline solution) phosphate buffer). The conjugation in the presence of glutaraldehyde may take place, e.g., according to the method described by S. Avrameas, Immunochemistry, Pergamon Press, 1969, 6, 43-52 [cited by N. Zegers. K. Gerritse, C. Deen, W. Boersma, and E. Classen, in J. Immunol. Meth., 130, 195 (1990)].

For instance, the glutharaldehyde method has been in particular adopted for the conversion of the following haptens into immunogens:
- sympathicomimetic drugs, which contain aromatic -NH₂ groups, such as cimaterol, clenbuterol;
- amino glycosidic antibiotics, e.g. neomycin, gentamicin, streptomycin;
- antimycotic drugs, e.g. amphotericin;
- amphetamine;
- antitubercolar drugs, e.g. cycloserine;
- sulphonylamides, e.g. sulphametoxazole.

### Method F - Conjugation of haptens containing thiol groups with a proteic carrier, previously functionalized with a disulfide reagent

Immunogens of haptens containing one or more thiol groups can be for instance obtained by conjugating them with proteic carriers, typically BSA, via a disulfide bridge, e.g. following the procedure described by D.A. Gadkari, H.A. Fields and J.E. Maynard, in J. Virol. Meth., 10. 215, (1985), or by T. Piao King, Y. Li, and L. Kochoumian, in Biochemistry, 17(8), 1499 (1978). Accordingly, haptens containing one or more -SH groups, for instance 2-thiouracils such as propylthiouracil, can be conjugated with a proteic carrier, typically BSA or HSA, which has been previously treated with a reagent able to functionalize the proteic carrier with disulfide bonds, i.e. a disulfide reagent, such as N-succinimyl-3-(2-pyridyildithio)propionate (SPDP) [i.e. 3-(2-pyridyldithio)propionic acid N-hydroxysuccinimide ester], which affords the corresponding 2-pyridyldithio-protein, or with 4,4'-dithio-dipyridine (generally upon amidination, e.g. with 2-iminothiolane), which affords the corresponding 4-pyridyldithio-protein.

Both functionalization of the proteic carrier with the disulfide reagent and conjugation of the hapten with the derivatized proteic carrier can be for instance carried out at room temperature (e.g. at 23°C), at a pH generally of 7.0-7.5 (e.g. in PBS buffer, pH 7.4).

Hapten is reacted with the proteic carrier, typically BSA, e.g. in molar ratios with respect to proteic carrier typically of from 15:1 to 50:1, e.g. 20:1 (essentially corresponding ratios are found in the conjugate).

The molar ratio between the dithio reagent and the protein can be for instance of about 3:1.

### Method G - Conjugation of haptens containing aromatic groups reactive towards diazonium salts, with a proteic carrier, in the presence of a bis-diazo reagent

This method is suitable for instance for haptens such as isoniazide. The carrier is typically BSA or HSA, and the bis-diazo derivative is for instance bis-diazotized benzidine. Reaction medium, temperature, pH, and hapten: proteic carrier molar ratios can be for instance as hereinafter described for method H. The procedure of R.M. Bassiri, R.D. Utiger, in Endocrinology, 90, 722 (1972) can be for instance followed.

### Method H - Conjugation of diazotized haptens with proteic carrier

Haptens containing primary amino groups, in particular aromatic ones, such as sulphonyl amides, can be diazotized and coupled with a proteic carrier, e.g. BSA, HSA or OVA, for instance HSA.

According to some particular embodiment conjugation of the diazotized hapten with the proteic carrier is carried out in an aqueous medium, e.g. at pH of about 9.0-10.0, at a temperature e.g. of about +2°C/+5°C.

Diazotized hapten: proteic carrier molar ratios used to prepare the product can be for instance of from 100:1 to 150 to:1.

An example is the azo-hapten-protein conjugate obtained by reaction of diazotized sulphomethoxazine with HSA, such as that according to Example 11c.

Drugs and metabolites thereof can be converted into immunogens not only according to the methods cited in the present text, but also by other methods known in the immunological field for the preparation of conjugates of haptens (polysaccharides, polypeptides, toxins or other substances) with proteic (e.g. BSA) or polysaccharide (e.g., dextrane having a MW of 4,400) carriers, which are known for diagnostic or immunization (e.g., vaccines) purposes.

### Production of IgG immunoglobulins

The immunogen derivatives obtained as described above are administered to the animals selected for the production of IgGs active as specific antibodies, according to conventional techniques typically by a primary injection followed by a booster. Animal serums are then extracted and the proteic fraction corresponding to IgG immunoglobulins is isolated therefrom and purified.

For commercial-scale production, the animals used are of medium/high size, such as goats and horses.

The claimed immunogen derivatives are preferably administered in combination with Freund's adjuvant. The method adopted is preferably that described by A. Johnstone and R. Thorpe, in Immunochemistry in Practice, p. 28, A. Johnstone and R. Thorpe, eds., Blackwell Sci. Publ., Oxford (1982).

In general, in the primary injection, the immunogen is combined with Freund's complete adjuvant (FCA) and, in the booster, with Freund's incomplete adjuvant (FIA). The primary injection is preferably administered by the intradermal route and the booster, typically administered 14 to 21 days later, by the intramuscular (i.m.) route. In both cases, each immunogen dose is generally of about 0.1 to 2 mg per kg of body weight, the first dose being preferably subdivided into several injections administered in different parts of the body.

IgG formation in the treated animal may be followed by passive hemoagglutination against the hapten covalently conjugated with turkey erythrocytes or by other conventional analytical methods.

About 30 to 90 days are required for the immunogen-treated animals to produce significantly high amounts of IgGs. After said period, a sample of blood is taken, generally from the femoral artery, wherefrom the proteic fraction containing IgG immunoglobulins is separated from serum, e.g., by precipitation by addition of ammonium sulphate.

The IgGs so obtained are preferably purified by ion-exchange chromatography, e.g. with matrix poly-[N-tris(hydroxymethyl) methylacrylamide functionalized with 2-(diethylamino)-ethyl groups, such as 1 M DEAE-Trysacryl (available from IBF), eluting with a buffer having a pH of 8.8. Typically, the cryoprecipitated serum containing IgGs is purified, and a preliminary passage on ULTROGEL AcA 202 column is performed for desalination.

The IgGs may be purified according to other conventional techniques, e.g, by affinity by immobilization chromatography on protein A.

### Conversion of IgG immunoglobulins into F(ab')₂ fragments

Fc fragments are removed by subjecting IgGs to digestion with pepsin, preferably immobilized, for instance under the solvent, pH and temperature conditions reported below.

Under typical conditions, the treatment with pepsin is carried out on proteic fractions precipitated by addition of polyethylene glycol, as disclosed by B. Favreau, D. Giurgiu, and B. Bizzini, in Experientia, 39, 483 (1983).

The process comprises of the following steps: precipitation of IgGs by treatment of the serum containing same with polyethylene glycol, typically at a pH of about 7.0 to 7.5, preferably of 7.0, at a temperature ranging from about 8°C to 25°C, e.g., at 20°C to 25°C; treatment of the resulting precipitate with pepsin, e.g., pepsin fixed on porous glass balls, at a pH of about 2.5 to 4.0, preferably of 3, at a temperature typically ranging from 25°C to 37°C, e.g., at about 30°C-32°C; precipitation of the fraction containing the F(ab')₂ fragments by addition of polyethylene glycol, typically at a pH of about 4.5 to 5.5, e.g., of 5, for instance at room temperature (e.g. +20°C/+25°C).

The F(ab')₂ fragments obtained are conveniently purified by dialysis, typically against 0.9% NaCl solution, overnight, at a temperature of 4°C to 10°C, e.g. at 4°C, and sterilized by filtration, usually through 0.45 µm filters.

### Diagnostic process

The claimed IgGs are used as diagnostic reagents in an Enzyme Linked Immunosorbed Assay (ELISA) of competitive type, e.g. analogous to that described by A. Voller, D.E. Bidwell, and A. Bartlett, in Bull. Wld. Health Org., p. 55, vol. 53 (1976).

Typically an inert solid support, is sensitized by fixation to IgG immunoglobulins active as antibodies to the compound to be proportioned, then added with the sample of biological fluid (e.g., serum, urine, milk) to be assayed, and then with a conjugate represented by the compound to be proportioned labelled with an enzyme, which is added either as a mixture with the sample to be assayed or a short time after the addition of said sample, after incubation, e.g. at 37°C, for about 30-60 minutes. The mixture is caused to react at 37°C for 30-60 minutes, preferably 60 minutes, to allow the fixation both of the compound to be proportioned and of the conjugate to the reactive site of IgGs; the mixture is washed and added with a chromogenic substrate which reacts with the labelling enzyme and, in the presence of the enzymatic portion of the conjugate, yields a coloured product. Upon colouring, the readout of the solution optical density is performed, in general directly in the proportioning microplate well, and the quantity of drug and/or metabolite contained in the assayed biological fluid is calculated.

Preferably, the enzyme is peroxidase, more particularly horse-radish peroxidase (HRP), and the chromogenic compound is orthophenylenediamine or tetramethylbenzydine (TMB), whose reaction with peroxidase is promoted by H₂O₂.

The drugs are. e.g., those listed herein for the claimed detoxifying treatment and/or as haptens for the preparation of immunogens.

The sample to be assayed is typically added in an amount sufficient for yielding a concentration of compounds to be assayed in a stoichiometric defect with respect to the IgG active sites: the compound to be proportioned and the enzyme-labelled compound compete for the linking sites of the specific antibody; the remaining sites are then bound to said conjugate, consequently part of the enzymatic activity is fixed to the solid support. The concentration of drug and/or metabolite to be proportioned is inversely proportional to the concentration of enzyme-labelled compound that is fixed to the antibody, and is calculated by difference, i.e., by substracting the optical density of a sample in which IgG immunoglobulins have been completely saturated with the enzyme-labelled compound from the optical density of the IgG sample treated with the assayed biological fluid.

The solid support (e.g. a plate of plastic material, such as polystyrene) is sensitized with the IgG in a conventional manner, e.g., by treatment with the IgGs, at a pH of about 7.0-7.5, e.g. with 4 µg/100 µl IgG in PBS buffer at a temperature ranging from 4°C to 37°C (e.g., at 4°C to 8°C, overnight, or at 37°C for about 2 to 6 h), the sites of the solid support which failed to react with the IgGs are then saturated by successive addition of casein (e.g. a 2% casein solution in PBS) e.g. at a pH of about 7.0-7.5. at about 4°C to room temperature (e.g. +20°C/+25°C).

The reaction of the IgGs fixed to the solid support with the solutions to be assayed (sample solutions, enzyme-labelled solutions containing the drug or the metabolite, standard reference solutions) is typically carried out by incubation at about 20°C to 37°C, e.g., at 37°C, and at a pH of about 7.0-7.4, for 30-60 minutes, typically 60 minutes. Said pH and temperature conditions may be adopted, e.g., also for colour development after chromogen addition, incubating for 30 minutes. As concerns washings and other operating details, reference is made to the Experimental Part.

### Diagnostic kit

The reagents useful for the diagnostic assay are advantageously assembled in an immunodiagnostic kit, to be directly used e.g.. by the farmer. Said kit comprises an IgG immunoglobulin supported on a solid phase, the conjugate, represented by the enzyme-labelled compound to be proportioned (drug or metabolite), and a chromogenic substrate.

The kit may be also provided with buffer solutions for sample dilution and with standard solutions containing predetermined concentrations of the compound to be assayed.

### Conjugates represented by the enzyme-labelled compound to be proportioned (drug or metabolite)

Said conjugates are obtained by condensation of the compound to be proportioned (drug or metabolite) with an enzyme, in particular a peroxidase, for instance according to the aforementioned methods of preparation of immunogen derivatives, wherein the hapten is the compound to be proportioned and the enzyme is the proteic substance to be used instead of the proteic carrier, or according to other known conjugation methods. For instance the compound to be proportioned can be reacted with the enzyme (peroxidase), in the presence of glutaraldehyde, or with the SPDP method, or as described by A. Voller, D.E. Bidwell and A. Bartlett, in Bull. Wld. Health Org., 53, 55 (1976) in the presence of a carbodiimide.

Examplary conjugates useful as diagnostic reagents are obtained by conjugation of:
a') a product obtained by conjugation of a hapten containing primary amino -NH₂ groups or -COOH groups, with an enzyme in the presence of a carbodiimide;
b') a product obtained by conjugation, in the presence of a carbodiimide, of a hapten containing primary amino -NH₂ groups, with an oxidized enzyme/synthetic poly-amino acid copolymer conjugate, wherein the carrier has been previously oxidized with an oxidizing agent of the -C-OH groups to C=0 groups;
c') a product obtained by conjugation of an enzyme with a hapten containing -C-OH groups, previously oxidized with an oxidizing agent of the -C-OH groups to C=O groups conjugation being followed by reduction with NaBH₄;
d') a product obtained by conjugation of a hapten containing primary amino -NH₂ groups, with an enzyme previously oxidized by treatment with an oxidizing agent of the -C-OH groups to C=0 groups conjugation being followed by reduction with NaBH₄;
e') a product obtained by conjugation of a hapten containing primary amino -NH₂ groups with an enzyme, in the presence of glutaraldehyde;
f') a product obtained by conjugation of a hapten containing one or more thiol groups with an enzyme previously functionalized with a disulfide reagent;
g') a product obtained by conjugation of a hapten containing one or more aromatic groups reactive towards diazonium salts, with an enzyme, in the presence of a bis-diazo reagent.
h') a product obtained by conjugation of a diazotized hapten with an enzyme.

Analogously with the considerations set forth for immunogens, the conjugates with enzymes of types a') and b') are particularly applied to drugs or metabolites containing -NH₂, or -COOH groups; the conjugates of types d') to compounds containing primary aliphatic or aromatic -NH₂ groups. The considerations made for the analogous immunogens, i.e. concerning solvent, pH, temperature, molar ratios between reagents, and/or the poly-amino acidic copolymer, hold good also for the preparation of conjugates a') to g').

the enzyme is typically horse radish peroxidase having a molecular weight, e.g., of about 45,000.

Exemplary drugs or metabolites are those mentioned above for the detoxifying treatment and/or as haptens for the preparation of immunogens.

The following examples are conveyed by way of indication, not of limitation, of the present invention, which is defined by the appended claims

### Method B - Conjugation of hapten, in the presence of carbodiimide, with a glycoprotein oxidized with periodate and conjugated with a poly-amino acid copolymer

a) 20 mg of proteic substance (glyco-proteic carrier for immunogen preparation or enzyme for drug/enzyme conjugates), having a MW of 40,000 to 200,000 Daltons, dissolved in 1.4 ml of 0.1 M acetate buffer, pH 6.5, was added with 200 µl of NaIO₄ 0.1 M dissolved in the same buffer immediately prior to use. The mixture, shielded from light, was caused to react for 30 min at room temperature with gentle stirring. The reaction was discontinued by addition of 400 µl of 1:1 glycerol/water, stirred for 5 min, allowed to stand for 4 h at 4°C.
b) Reaction of oxidized protein and poly-(D-Glu,D-Lys) copolymer, Sigma ^{R} P7658 (having a MW of 50 KD); D-Glu:D-Lys ratio = 6:4; poly-(D-Glu,D-Lys):oxidized protein molar ratio equal to 1:15.
About 1.466 mg poly-(D-Glu,D-Lys) were necessary for 20 mg protein. The reagents used were:

| | |
|---|---|
| protein 20 mg in | 2,000 µl acetate buffer |
| 0.1M CO₃⁻⁻/HCO₃⁻ buffer, pH 9.0 | 200 µl |
| Poly-(D-Glu,D-Lys) (2 mg/ml) in | 733 µl carbonate buffer |
| 0,1M CO₃⁻⁻/HCO₃⁻ buffer, pH 9.0 | 267 µl |

0.1 M carbonate buffer was added by adjustment of the reaction volume. The mixture, shielded from light, was caused to react at 37°C for 4 h. under stirring. Then at the end of reaction it was treated with 465 µl of NaBH₄ (2 mg/136 µl of H₂O), allowed to stand at 4°C for 4 h, still shielded from light. The resulting mixture was concentrated by centrifugation (3,000 rpm (rounds per minutes)) for 30 min, on 100 KD microfilter Amicon. A waste volume of about 1.6 ml containing free (non-conjugated) protein was obtained. An equal volume of buffer was added to the concentrate. The product was subjected to dialysis on 100 KD - 200 KD molecular cut-off tube, depending on the carrier M.W., against 0.1 M acetate buffer, at a pH of 5.5. Four dialysis passages in the cold for a total of about 30 h were performed.

### c) Conjugation with the hapten

15 to 50 mol of hapten per mol of poly-amino acidic copolymer having a MW of 50 KD was used.

A solution of oxidized protein/poly-(D-Glu,D-Lys) copolymer conjugate (having a MW of 50 KD), in a poly-(D-Glu,D-Lys) copolymer concentration of 8 to 10 µM/ml (micromoles/milliliter) in acetate buffer 0.1M pH 5.5, and hapten solutions, in concentrations of 30 to 100 µM/ml, in acetate buffer 0.1M pH 5.5 and optionally solvents to solubilize, were prepared. The following ingredients were mixed:
- a solution of oxidized protein/poly-(D-Glu,D-Lys) copolymer conjugate in acetate buffer 1.0 ml
- 0.1 M acetate buffer, pH 5.5 1.5 ml
- hapten solution in acetate buffer 1.0 ml

The solution was added with 65 mg of N-ethyl-N'-(3-dimethylaminopropyl)carbodiimide (10 mg/10 µl in water CDI solution to be prepared immediately prior to use). The reaction was continued for 30 min at 37°C. The resulting product was dialyzed against PBS buffer (physiological solution-phosphate buffer) at a pH of 7.4 on a 30 KD molecular cut-off tube. The dialyzate was kept in a refrigerator.

### Method D - Conjugation of hapten with primary -NH₂ groups with a periodate-oxidized proteic substance

Reagents: PBS; 1M carbonate/bicarbonate buffer, pH 9.5; 0.1 M sodium periodate (21 mg/ml); ethylene glycol (1 ml ethylene glycol in 17 ml distilled water); sodium boron hydride (4 mg/ml distilled water solution, prepared immediately prior to use).

Procedure: 2 mg of proteic substance in 0.5 ml of 0.1 M sodium periodate was dissolved in 0.1 M acetate buffer, pH 5.5. The solution was stirred at room temperature for 20 min, added with 30 µl of 1 M glycerol to block the action of excess periodate, stirred for additional 5 min. filtered through fine Sephadex G 25 column (1x5. equilibrated with 0.1 M carbonate/bicarbonate buffer, pH 9.5).

A brown fraction was collected, which provides a O.D. read in UV at 280 nm. 10 mg of hapten in little saline physiological solution (about 1 ml) and then 0.1 ml of 1 M carbonate/bicarbonate buffer were added.

After 2-h stirring at room temperature, 0.2 ml of 4 mg/ml sodium boron hydride was added. The resulting solution was allowed to stand for 2 h, then dialyzed against PBS overnight, centrifuged for 15 min (10,000 rpm), and added with an equivalent volume of glycerol.

### Method E - Conjugation of hapten with protein in the presence of glutaraldehyde

10 mg of proteic substance was dissolved in 1 ml of 0.1 M phosphate buffer at a pH of 6.8, wherein the hapten, in a proteic substance/hapten molar ratio equal to 1:15, had been dissolved. The solution was added with 50 µl of 1% glutaraldehyde in 0.1 M phosphate buffer at a pH of 6.8, caused to react at room temperature for 3 h, added with 50 µl of lysine 1M pH₇ (pH 6.6-7.4) in PBS to block the glutaraldehyde reactive groups. The resulting product was allowed to stand at room temperature for 1-2 h or overnight in a refrigerator, and then dialyzed against PBS buffer on a 10 KD molecular cut-off tube, overnight, centrifuged for 20 min (10,000 rpm), added with an equivalent volume of glycerol, and kept at a temperature ranging from 4°C to -20°C.

### Method C - Conjugation of periodate-oxidized hapten with proteic substance

² mg of hapten with glucydic -OH groups were dissolved in 0.5 ml of 0.1 M NaIO₄ dissolved in 0.1 M acetate buffer, pH 5.5. The solution was allowed to stir at room temperature for 20 min, added with 30 µl of 1 M glycerol, stirred for additional 30 min, cooled to 4°C for about 1 h and a half, added with NaOH to a pH of about 9.0 and with 0.1 M carbonate/bicarbonate buffer until obtaining a final volume of 1 ml, containing 8.0 mg of proteic substance. The resulting solution was stirred at room temperature for 2 h, added with 200 µl of 4 mg/ml sodium boron hydride, allowed to stir at room temperature for 2 h, and dialyzed against physiological solution.

### Method F -Conjugation with a proteic carrier through disulfide bonds (SPDP method)

Reaction Scheme for haptens with -SH groups (e.g. propylthiouracil): A solution of 1 mg/ml of BSA (M.W. 65.000 D) in PBS buffer (0.1M, pH 7.4), cooled with ice, is added under magnetical stirring with 5 µl of a 10 mM ethanolic solution of N-succynimyl-3-(2-pyridilthio)-propionate (SPDP). The reaction mixture is reacted at room temperature (e.g. 23°C) for 30-60 minutes, e.g. 30 minutes, under stirring at a low rate, avoiding foam formation. At the end of reaction, the solution is immediately filtered through a sephadex column 25 (1x10 cm) with phosphate buffer 0.1M, pH 7.4.

The fractional containing the 2-pyridyldisulfide derivative are collected as 1 ml fractions, and stored at 4°C under nitrogen, or used immediately afterwards.

Hapten is reacted in a molar ratio of from 15:1 to 50:1, e.g. 20:1, with respect to BSA, 1 ml of solution containing the BSA-S-S-2-pyridyl derivative is added with 2 ml of a hapten solution in PBS, in the aforementioned molar ratios, and reacted at room temperature for 30-120 minutes, e.g. 60 minutes, affording the conjugate product in a 70% yield. The conjugate can be directly used as specific immunogen for production of antibodies, without any purification.

### PREPARATION OF IMMUNOGENS

**Example 1A - Immunogen derivative of haptens conjugated with a proteic carrier represented by the oxidized gamma globulin/poly-(D-Glu,D-Lys) copolymer conjugate**

Method B was repeated using gamma-globulin IgG having a MW of 160,000 as a protein, each time using one of the following haptens: neomycin, gentamicin, streptomycin, amphotericin, cycloserine, amphetamine, sulphamethoxazole. **Example 1B - Immunogen of tetracyclines**

Oxytetracycline and doxycycline were derivatized by treatment with diazotized para-amino benzoic acid, then conjugated with bovine serum albumin (BSA having a MW of 65,000) in the presence of N-ethyl-N'-(3-dimethylaminopropyl)-carbodiimide according to the process described by E.C. Beuvery, G.J.A. Speijers, B.I.G. Lutz, D. Freudenthal, V. Kanhai, B. Haagmans, and H.J.G.M. Derks, in Develop. Biol. Standard, S. Karger, Basel (1986), 63, 117.

### Example 2 - Immunogen of amino glycosidic antibiotics

Method C was repeated using BSA having a MW of 65,000 as a protein, and each time using as a hapten one of the following compounds: neomycin B, gentamicin, streptomycin, amphotericin and idoxuridine.

### Example 3 - Immunogens of BSA-conjugated drugs in the presence of glutaraldehyde

Method E was repeated using BSA having a MW of 65,000 as a proteic substance, and each time using as a hapten one of the following compounds: neomycin, gentamicin, streptomycin, amphotericin; cycloserine; amphetamine, sulphamethoxazole.

### Example 4 - Immunogen of cephalosporins

Ceftazidime, cefotaxime, cephalexin were treated with bovine serum albumin (BSA, having a MW of 65,000), in the presence of a carbodiimide as a conjugation reagent, operating under the conditions described by the reference of E.C. Beuvery, reported in Example 1B.

### Example 5 - Immunogens of penicillins 6-aminopenicillanic acid and penicilloic acid were treated with bovine serum albumin, according to the direct carbodiimide conjugation method. Example 6 - Immunogen of sulphonamides

Sulphamethoxazole [3-sulphanylamido-5-methylisoxazole] was conjugated with gamma-globulin/copolymer in the presence of a carbodiimide, according to method B. **Example 7 - Immunogen of anti-thyroid 2-thiouracyl derivatives**

Propylthiouracyl was conjugated with BSA in the presence of SPDP, under the operating conditions above described. **Example 8 - Immunogens of diethylstilbestrol [3,4-bis-(p-hydroxyphenyl)-3-hexene], 17-β-estradiol, zeranol [lactone of 6-(6,10-dihydroxyundecyl)β-resorcinicol acid], trenbolone [17-β-hydroxy-estra-4,9,11-trien-3-one]**

Said immunogens were obtained conjugated the drugs of the title, previously derivatized as above described in the present text, with BSA, in the presence of a carbodiimide, according to method A.

### Example 9 - Immunogen of olaquindox (OLA)

### - 9a - Preparation of diazotized PABA

p-Aminobenzoic acid (PABA) (8.2 mg; 6 x 10⁻⁴ moles) was dissolved in 1N HCl (6 ml). The solution was ice cooled and added with NaBr (10 mg). A NaNO₂ (37 mg; 5.4 x 10 ⁻⁴ moles) solution in H₂O (2ml) was extemporarily prepared, ice cooled and added dropwise under stirring in 10 minutes to the PABA solution. Stirring was continued for one additional hour at 4°C.

### - 9b- Preparation of olaquindox-PABA azoderivative

OLA (142 mg; 5.4 x 10⁻⁴ mole) was dissolved in 2N NaOH₄ (about 6 ml), adjusting the volume to 10 ml with water. This solution was ice cooled, then added with diazotized PABA at small portions, smaller than drops. During the diazotized PABA addition pH was adjusted to 9-10 by 2N NaOH addition. At the end of diazotized PABA addition, pH is adjusted to 9 and the solution is stirred in ice for additional 4 hours.

### - 9c - Preparation of OLA-OVA conjugate (control)

Conjugation was carried out by reacting chicken ovoalbumin (OVA) (4 ml of a 10mg/ml solution = 40 mg) with the diazo-OLA derivative solution (8 ml) (pH= 6.0) in the presence of carbodiimide, for 15 hours at the laboratory temperature (at about + 10°C). At the end of reaction, the excess of carbodiimide was removed by dialysis against saline solution.

Molar ratios OVA-OLA :
8 ml azo-OLA : 2.16 x 10 ⁻⁴ mole azo-OLA
4 ml OVA : 40 mg : 1 x 10⁻⁶ mole OVA
Molar ratio diazo-OLA:OVA = 200 :1

### - 9d - Preparation of OLA-HSA conjugate

The reaction was carried out under the same conditions used for the OLA-OVA conjugate, by using HSA (4 ml solution containing 40 mg; 6 x 10⁻⁷ mole of HSA), azo-OLA derivative solution (4ml); carbodiimide 100 mg.

Molar ratios HSA-OLA (Immunogen):
4 ml azo-OLA : 1.08 x 10 ⁻⁴ mole azo-OLA
4 ml HSA : 40 mg : 6 x 10⁻⁷ HSA
Molar ratio diazo-OLA:HSA = 166 :1

The OLA-HSA conjugate was injected in animals to produce antibodies as described below, affording IgG in yields of 25.3 mg/ml.

The OLA-OVA conjugate was used as control in the ELISA test hereinafter described to test the antibody specificity: an antibody specific for a given hapten gives a response both with the injected immunogen and with a conjugate of the same hapten to a different proteic carrier.

### Example 10 - Immunogen of oxytetracycline (OTC)

OTC 1 mg = 2.17 x 10⁻⁶ moles

In this and in the above Examples the carbodiimide was N-ethyl-N'-(3-dimethylaminopropyl)-carbodiimide.

### - 10a - Preparation of diazotized PABA

Diazotized PABA was prepared according to Example 9a using PABA (41 mg), 1N HCl (3 ml), NaBr (5 mg), NaNO₂ (18.5 mg).

Final volume : 4.9 ml.

### - 10b - Preparation of OTC-PABA azoderivative

OTC (100 mg) was dissolved in 2N NaOH (2 ml), adjusting the volume to 5 ml with water. The OTC solution (2.17 x 10⁻⁴ moles) was added with the diazotized PABA solution (3.2 ml; 1.96 x 10⁻⁴ moles) and treated as previously described for Example 9b.

Final volume : 8.2 ml.

### - 10c - Preparation of OTC/OVA conjugate (control)

OTC was conjugated with OVA under the same reaction conditions of Example 9c.

The following amounts of OVA and OTC were used:
OVa : 4 ml of a solution containing 40 mg (1 x 10⁻⁶ moles)
azo-OTC : 2 ml containing 5.28 x 10⁻⁵ mole
carbodiimide : 100 mg
Molar ratio OTC : OVA = 52.8 : 1 = about 53

### - 10d - Preparation of OTC-HSA conjugate

OTC was conjugated with HSA under the same reaction conditions of Example 9d.

The following amounts of HSA and OTC were used:
HSA : 4 ml of a solution containing 6 x 10⁻⁷ moles
azo-OTC : 3 ml containing 7.92 x 10⁻⁵ mole
carbodiimide : 100 mg
Molar ratio OTC : HSA = 792 : 6 = 132
After immunization of animals by injecting the OTC.HSA conjugate, IgG were obtained in a yield of 27.07 mg/ml.

As above reported, the OTC/OVA conjugate was used in the ELISA test as control.

### Example 11 - Immunogen of sulfamethazine (SM)

### - 11 a - Preparation of diazotized SM

SM : 1 mg (3.59 x 10⁻⁶ moles)

SM (25 mg; 0.9 x 10⁻⁴ mole) was dissolved in conc. HC1 (2 ml) + H₂O (2 ml), the resulting solution was ice cooled then added with NaBr (2.5 mg).

The SM solution thus obtained was added dropwise in 10 minutes under stirring, under ice cooling, with a solution of NaN0₂ in H₂O (2 ml). The resulting mixture was stirred for one additional hour, under ice. Then the volume was adjusted to 12 ml.

### - 11b - Preparation of SM-AZO-OVA conjugate (control)

OVA : 4 ml containing 40 mg: 1 x 10⁻⁶ mole

Diazo-SM : 3 ml containing 7.5 x 10⁻⁶ mole x 3 = 2.25 x 10⁻⁵ mole The solution containing the diazotized-SM was added dropwise, under stirring, in 15 minutes, to the OVA solution, maintaining the pH at about 9.0-10.0 by 2N NaOH addition. At the end of addition, the reaction mixture is kept under stirring for additional 4 hours at 4°C. The conjugate solution is dialyzed against saline solution. Molar ratio SM : OVA = 22

### - 11c - Preparation of SM-AZO-HSA conjugate

Diazo-SM was conjugated with HSA under the same reaction conditions of Example 11b.

The following amounts of HSA and Diazo-SM were used:
HSA : 2 ml of a solution containing 20 mg; 3.0 x 10⁻⁷ moles
diazo-SM : 4.5 ml containing 3.37 x 10⁻⁵ mole
Molar ratio SM : HSA = 112
After immunization of animals by injecting the SM-conjugate yields of specific IgG of about 30.2 mg/ml were obtained.

Analogously to what above reported, the SM-OVA conjugate was used as control in the ELISA test.

### Example 12 - Production of specific IgG antibodies to immunogens

A. Production of antibodies. The immunogens prepared as described in the examples reported above were administered to goats or horses, in the presence of Freund's adjuvant, according to the scheme described by A. Johnstone and R. Thorpe, in Immunochemistry in Practice, p. 28, A. Johnstone and R. Thorpe, eds., Blackwell Sci. Publ., Oxford (1982), more particularly following the process described below:
**Primary immunization:** The animals were given an oil/water emulsion containing the immunogen (0.1-2 mg per kg of body weight) in PBS (2 ml), emulsified with FcA (Freund's complete adjuvant) in the presence of an emulsifier, by subdividing the dose into several injections administered intradermally in different parts of the body.

Booster: 14 to 21 days after the primary injection, goats or horses were given a preparation containing the immunogen (0.1-2 mg) in PBS (2 ml) and FIA (Freund's incomplete adjuvant), by intramuscular route. The immune response was checked as described hereinafter on a blood sample taken from the femoral artery, 20 to 40 days after booster administration.

The serum containing IgGs was withdrawn and used to prepare immunodetoxifiers consisting of the F(ab')₂ fraction of the antibody and to purify the IgGs to be used in the diagnostic test

B. Purification of IgGs from the serum. Said purification was carried out by chromatography on 1 M DEAE-Trysacryl, eluting with 0.025 M Trys-HCl (0.035 M/NaCl) buffer, having a pH of 8.8. The immunoglobulins of class IgG, being 98-99.5% pure, were eluted, whereas those belonging to the other classes were retained. Chromatography on DEAE-Trysacryl column was conducted on plasma cryoprecipitated to remove, e.g.. fibrinogen, chylomicrons, lipoproteins, whereby the chromatographic columns might be damaged, and was preceded by a passage on ULTROGEL AcA 202 column, eluting with the aforesaid buffer, for desalination.

The operating process is described, e.g., in the technical bulletin provided by the supplier: "Reactifs IBF". "Preparation of Albumin and IgGs by Direct Chromatographic Separation of DEAE- and CM-Trysacryl M", by J. Saint-Blancard. J. Fourcart, E. Boschetti, P. Girot. Once chromatography had been performed, IgGs were concentrated again to the original volume on 10 KD molecular cut-off microfilter Amicon and dialyzed for 24 h against physiological saline solution.

Titration of the proteic content was carried out by the biuret method. The antibody activity was evaluated according to ELISA, an immunoenzymatic method of competitive type, according to which the hapten is bound to the inert support, and the antibody concentration is detected by means of a anti-antibody-enzyme (HRP) conjugate or to the precipitin method or other conventional methods.

### C. Proportioning of IgGs in the serum of the IgG-producing animal:

IgGs were determined quantitatively by conventional methods, e.g., by passive hemoagglutination against the hapten conjugated covalently with sheep erythrocytes, (E.H. Relyveld, P. Saliou and N.Le Camp, L'eurobiologiste, 1990, tome XXIV. No.187, Pag. 219-228) titrating the IgG antibody present in the sample, if any, in wells added with erythrocytes sensitized through linking with the hapten. An opaque and homogeneous well surface indicated the presence of antibodies in the assayed sample.

IgGs dosage on microplate with "V" wells, on serum, plasma or whole blood, as follows:
1. 1:20 dilution in PBS of a sample (animal serum containing the IgGs);
2. Buffer distribution (50 µl) into wells;
3. Addition to wells or diluted samples (50 µl)
4. Addition to well of 50 µl sensitized erythrocytes;
5. 1 minute stirring
6. Results readout: positive ones = homogeneous opaque well surface; negative ones = big dense preicpitate at the well bottom; weakly positive = small precipitate at the well bottom.

Samples can be quali-quantitatively evaluated, comparing samples with standard reference preparations serially diluted to known concentration.

### Example 13 - Preparation of F(ab')₂ fragments

For 1000 ml serum. The serum pH was adjusted to about 7 (e.g. 7.4). The serum was slowly added at room temperature with 1000 ml of 32% PEG_{6000'} in water, having a pH of 7 (final PEG 16%) and precipitated for at least 30 min at room temperature. Precipitate PI was recovered by filtration through Laurent's filter No. 1. The filtrate was discarded. Precipitate PI was dissolved in 1.500 ml of 0.9% NaCl (3/4 of 2,000 ml, i.e.. 3/4 of the volume of the first precipitation). The pH was adjusted to 7. The solution was added slowly with 1,500 ml (v/v) of 28% PEG₆₀₀₀ solution [final PEG 14%] and precipitated for at least 30 min at room temperature. Precipitate PII was recovered by filtration. The filtrate was removed. Precipitate PII was dissolved in 1,000 ml of 0.9% NaCl and added with 16.5 g of 0.22 M glycine. After adjusting the pH to 3 with 1N HCl, the solution was placed on water bath at 31.5°C.

When the temperature reached the desired value, the solution was added with 200.000 U pepsin (200 U/ml PII), left on water bath at 31.5°C for 90 min, and stirred at 15-min intervals. The pepsin dregs were eliminated by 10-15 minutes' contrifugation (3.000 rpm).

The supernatant concentration was adjusted to 0.5 M NaCl from the initial concentration of 0.15 M, by addition of 20 g NaCl. The pH was adjusted to 7.5 (pepsin neutralization) and then to 5. The solution was added with 350 ml of 40% PEG₆₀₀₀ [final PEG 10%], precipitated for at least 30 min at room temperature. The filtrate, SIId, was recovered. The optical density (OD) was read at 280 nm (an aliquot of the filtrate was diluted 1:20 to evaluate protein concentration). The pH was adjusted to 7 by addition of 1N NaOH. Some time later, 900 ml (equal volume) of 50% PEG₆₀₀₀ was added (final PEG = 30%). The resulting solution was precipitated for at least 30 min at room temperature.

The precipitate obtained was recovered, dissolved in the smallest quantity required of 0.9% NaCl (200 ml) and dialyzed against 0.9% NaCl (2 litres), overnight, at 4°C. The dialyzate was recovered, contrifuged for 15 min (4,000 rpm). The optical density was measured and the concentration of F(ab')₂ was adjusted to 10 mg/ml.

The product obtained was filtered through 0.45 µm and successively 0.2 µm filters and distributed in tubes (20 mg/2ml/tube). A sterility test (100 µl Fab')₂ + 0.9 ml of medium with serum without antibiotic) was performed.

### Example 14 - ELISA-type competitive diagnostic assay for the determination of circulating drugs

The method was analogous to that described by A. Voller, D.E. Bidwell, and A. Bartlett, in Bull. Wld. Health Org., 53, 55 (1976).

A. Adhesion of IgG antibody to the solid phase. A purified IgG solution in 1 M carbonate buffer, pH 9.5, with 50 µg/ml protein content, determined by the biuret method, was prepared. Each well of a polystyrene microplate was added with 100 µl solution and incubated at 37°C for 2 h; then it was added with 100 µl of a 2% (w/v) casein solution in 0.05 M PBS buffer, at pH 7.4. and allowed to saturate overnight in a refrigerator. Casein was removed and the wells were washed three times with PBS buffer. The microplate was dried in a desiccator with silica gel, under vacuum.

B. Sample solution: animal serum to be checked.

C. Enzyme-labelled compound to be proportioned: the compounds to be proportioned (haptens) were linked to horse-peroxidase (HRP) by different methods according to their structure.

### Conjugates wherein the drug is labelled with oxidized peroxidase (HRP)/poly-(D-Glu, D-Lys) copolymer conjugate

Method B was repeated using HRP having a MW of 45,000 as the protein. Conjugation was each time carried out with the haptens specified for immunogens preparation according to Method B in Example 1A.

### Conjugates of drug or metabolite containing -NH₂ groups and peroxidase (HRP) in the presence of glutaraldehyde

Method E was repeated using HRP having a MW of 45,000 as a protein and, as haptens, the haptens used for immunogens preparation according to method E in Example 3.

### Conjugates of aminoglycosidic antibiotics and HRP

Method C was repeated using HRP having a MW of 45.000 as a protein and, as haptens, the haptens used for immunogens preparation according to method C in Example 2.

### Immunodiagnostic assay

Reagents and instrumentation: The diagnostic kit includes:
1. Solid phase: polystyrene 96-well micro titrating plate (12 eight-well rows) incorporating the already adsorbed antibody specific for the drug to be proportioned.
2. PBS/casein diluting buffer (2 x 50 ml).
3. PBS/Tween concentrated wash solution (1 x 50 ml).
4. Conjugate (enzyme-labelled hapten): peroxidase-labelled haptens (1 x 320 µl).
5. Chromogen: TMB (tetramethylbenzydine) buffered solution (1 x 6 ml).
6. Substrate: H₂O₂ buffered solution (1 x 6 ml) [enzyme substrate].
7. Reagent to stop the reaction: 0.5-2N sulphuric acid (4 x 100 µl).
8. Positive controls: 8a. Standard 1; 8b. Standard 2; 8c. Standard 3; 8d. Standard 4 (the standard used is the diluted serum of animal treated with the drug to be proportioned, whose concentration has been determined by another analytical method known for said drug. It is thus possible to determine the various forms of the circulating drug or metabolite, be it free and/or linked to proteins and to prepare a calibration curve of drug or metabolite hematic levels.
   In some cases, positive controls may be represented by milk.
9. Negative control: (1 x 100 µl) (serum of untreated animal).

All reagents and the plate are to be kept at 2 to 8°C, shielded from light. The film covering the rows is to be removed immediately prior to use. Reagents are to be used after heating to room temperature.

Procedure: The dilutions indicated below are to be made immediately prior to use:
Dilute reagent 4 with reagent 2 (1:40; e.g. from 25 µl to 1 ml for each 8-well row).
Dilute reagent 5 with an equal volume of reagent 6 (e.g. 1 ml + 1 ml for each 8-well row).
Dilute reagent 3 with physiological solution (1:10).

Samples to be assayed: if the assay is not performed during the day of sample withdrawal, the sample is to be frozen at -20°C.

Serums inactivated at 56°C shall not be used.

Samples: dilute 1:100 with reagent 2.

Controls 8a, 8b, 8d, 9: dilute 1:100 with reagent 2.

Assays are carried out in duplicate on two well rows, by adding each well with 100 µl of the reagents indicated in Table 1, according to the scheme reported therein:

**Table 1**

| | Row No. 1 | Row No. 2 |
|---|---|---|
| 1 | W | Pd |
| 2 | W | Pd |
| 3 | Pa | N |
| 4 | Pa | N |
| 5 | Pb | S |
| 6 | Pb | S |
| 7 | Pc | - |
| 8 | Pc | - |

W = White (diluting buffer-reagent 2); Pa, Pb, Pc, Pd = Positive controls corresponding to dilute reagents 8a, 8b, 8c, 8d); N = Negative control (dilute reagent 9); S = Sample under examination, in the dilute form.

Rows are covered with an adhesive film and are incubated at 37°C for 1 h. Wells are washed three times with 350 µl wash solution (reagent 3). Once all wash residues have been carefully eliminated, each well is added with 100 µl of reagents in the following order:
- conjugate (reagent 4)
- chromogenic substrate (reagent 5).

After each addition of reagent, the row is covered, incubated at 37°C for 1 h. Before being added with the successive reagent, the wells are washed as specified above.

The reaction is stopped by addition of reagent 7 (50 ul/well). Mixing is carried out by a plate agitator, the optical density (OD) is read at 450 nm within 30 min, the zero value having been set as White. The assay is considered valid when the mean value of the negative control is E_{(negative)}≤ 0.100.

**Qualitative determination:** The positivity cutoff value ranges from 0.250 ≤ E(_{cutoff}) ≤ 0.350. The samples giving results below the cutoff value are "negative", whereas the samples giving results above the cutoff value are "positive". The determination is to be repeated with results ranging within the cutoff value interval.

**Quantitative determination:** The kit is provided with 4 standard serum dilution allowing the plotting of a calibration curve. To calculate the hapten concentration (ug/ml) in the sample, it is enough to extrapolate the OD value of the sample from the calibration curve previously plotted.

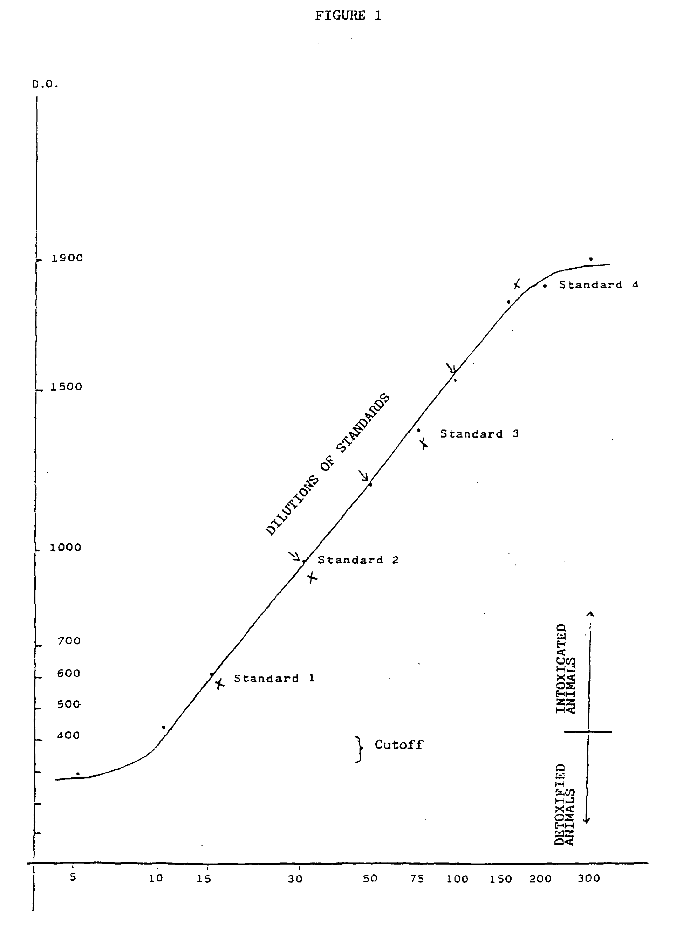

By way of example. Fig. 1 shows a calibration curve reporting the drug concentration of standard solutions 1, 2, 3, and 4, expressed as Log ug/ml on abscissa, and the OD on ordinate. The drug concentration that may be determined in the animal varies from ng to µg.

### Example 15 - Use of F(ab')₂ fragments for the detoxification from drugs

Bovine animals were subjected to treatment with the drugs (whose immunogens were prepared as described above), for instance neomycin, sulfamethoxazole, propylthiouracil, zeranol, cycloserin, amphetamine, olaquindox, oxytetracycline, sulfamethazine for a time securing a pharmacological effect. The drug or metabolite concentration in the serum was determined by the aforementioned competitive ELISA assay, calculating the drug concentration in the serum by the calibration curve obtained as reported above for ELISA assay.

Before detoxification, 100% positive values were observed (OD above the cutoff value ranging from 0.250 to 0.350; cf. Fig. 1). The drug concentration in the serum was determined on the basis of the calibration curve. The animals to be treated were then administered, by the i.m. route, 1 mol of F (ab')₂ specific for said drug/2 mols of drug or metabolite present in the serum.

The determination of the drug concentration in the serum by ELISA assay was repeated 48-72 h from F(ab')₂ administration. The detoxifying treatment was effective when the drug values in the serum were negative, i.g., below the OD cutoff value, as defined above. If positive values were still found, the detoxifying treatment was repeated by administering 1 mol F(ab')₂/2 mol drug.

The drug level in the blood was substantially reduced below the aforesaid cutoff value by the aforementioned detoxifying treatment, after a number of administrations generally ranging from 1 to 3.

## Claims

1. Use of a F(ab')2 fragment active as specific antibodies to a drug selected from the group consisting of: antibiotic, sulphonamide, antiviral drug, antiparasitic drug, antimycotic drug, antitubercular drug, sympathicomimetic drug, anti-thyroid agent, estrogenic, androgenic or progestinic substances, cortisone and corticosteroid analogs, endogenous hormone, auxinic drug, amphetamine growth stimulant, for the preparation of a medicament for the detoxification of a drug-treated animal by a intramuscular

2. Use as claimed in claim 1, wherein the antibiotic is selected among tetracyclines, amino glycosidic antibiotics, cephalosporins and penicillins; the sympathicomimetic drug is a β-agonist having 2-phenylethylamine structure; the anti-thyroid agent is a 2-thiouracyl derivative; the sulphonamide is substituted at the sulphonamide nitrogen with an isoxazole ring; growth stimulant has a quinoline structure.

3. Use as claimed in claim 1, wherein the antibiotic is selected out of oxytetracycline, doxycycline, neomicyn B, gentamicin C, tobramycin, kanamycin, amikacin, and streptomycin, ceftazidime, cefotaxime, cephalexin, a penicillin; the sulphonamide is sulphamethoxazole or sulphamethoxazine; the antiviral drug is idoxuridine; the antimycotic drug is amphotericin or ketoconazole; the antitubercular drug is cycloserine or isoniazid; the sympathicomimetic drug is clenbuterol or cimaterol; the anti-thyroid agent is methylthiouracil or propylthiouracil; the estrogenic substance is diethylstilbestrol, 17-β-estradiol or zeranolol; the androgenic substance is trenbolone or 19-nortestosterone; the progestinic substance is progesterone; the corticosteroid analog substance is dexamethasone; the endogenous hormone is somatotrophin; the auxinic drug is selected out of virginiamycin, zincobacitracin, avoparcin; the amphetaminic drug is amphetamine or methamphetamine, the growth stimulant is olaquindox.

4. Use as claimed in claims 1-3, wherein the F(ab')2: drug molar ratio is comprised from 1:1 to 1:5.

5. Use as claimed in claim 1-3, wherein said detoxification is performed before slaughtering or milk collection.

## Patentansprüche

1. Verwendung eines F(ab')2 Fragments, welches als spezifischer Antikörper gegen einen Wirkstoff aktiv ist, welcher aus der Gruppe ausgewählt ist bestehend aus: Antibiotikum, Sulfonamid, antiviralem Wirkstoff, antiparasitischem Wirkstoff, antimykotischem Wirkstoff, Antituberkulosewirkstoff, sympathikomimetischem Wirkstoff, antithyroidalem Wirkstoff, östrogener Substanz, androgener Substanz oder progestiner Substanz, Kortison- und Kortikosteroidanaloga, endogenem Hormon, Auxin-ähnlichem Wirkstoff, Amphetamin-Wachstumsstimulans für die Herstellung eines Medikaments zur Detoxifizierung eines mit einem Wirkstoff behandelten Tieres über den intramuskulären Verabreichungsweg.

2. Verwendung gemäß Anspruch 1, wobei das Antibiotikum ausgewählt ist unter Tetracyclinen, Aminoglykosid-Antibiotika, Cepahlosporinen und Penicillinen; bei dem sympathikomimetischen Wirkstoff handelt es sich um einen Beta-Agonisten mit einer 2-Phenylethylamin-Struktur; bei dem antithyroidalen Wirkstoff handelt es sich um ein 2-Thiouracil-Derivat; bei dem Sulfonamid ist der Sulfonamid-Stickstoff gegen einen Isoxalolring ausgetauscht; das Wachstumsstimulans weist eine Chinolinstruktur auf.

3. Verwendung gemäß Anspruch 1, wobei das Antibiotikum ausgewählt ist unter Oxytetracyclin, Doxycyclin, Neomycin B, Gentamicin C, Tobramicin, Canamycin, Amikain und Stretomycin, Ceftazidim, Cefotaxim, Cephalexin, einem Penicillin; bei dem Sulfonamid handelt es sich um Sulfamethoxazol oder Sulfamethoxazin; bei dem antiviralen Wirkstoff handelt es sich um Idoxuridin; bei dem antimykotischen Wirkstoff handelt es sich um Amphotericin oder Ketoconazol; bei dem antituberkulären Wirkstoff handelt es sich um Cycloserin oder Isoniazid; bei dem sympathikomimetischen Wirkstoff handelt es sich um Clenbuterol oder Cimaterol; bei dem antthyroidalen Wirkstoff handelt es sich um Methylthiouracil oder Propylthiouracil, bei der östrogenen Substanz handelt es sich um Diethylstilbestrol, 17-β-Östradiol oder Zeranolol; bei der androgenen Substanz handelt es sich um Trenbolon oder 19-Nortestosteron; bei der progestinen Substanz handelt es sich um Progesteron; bei dem Kortikoidanalog handelt es sich um Dexamethason; bei dem endogenen Hormon handelt es sich um Somatrophin; der Auxin-ähnliche Wirkstoff ist ausgewählt unter Virginiamycin, Zinkobacitracin, Avoparcin; bei der wie ein Amphetamin wirkenden Substanz handelt es sich um Amphetamin oder Methamphetamin; bei dem Wachstumsstimulans handelt es sich um Olaquindox.

4. Verwendung gemäß Ansprüchen 1-3, wobei das molare Verhältnis von F(ab')2/Wirkstoff 1:1 bis 1 :5 umfasst.

5. Verwendung gemäß Ansprüchen 1-3, wobei die genannte Entgiftung vor dem Schlachten oder der Milchabnahme durchgeführt wird.

## Revendications

1. Utilisation d'un fragment F(ab')2 actif en tant qu'anticorps spécifique dirigé contre un médicament choisi dans le groupe constitué de : antibiotique, sulfamide, médicament antiviral, médicament antiparasitaire, médicament antimycotique, médicament antituberculeux, médicament sympathicomimétique, médicament antithyroïdien, substances oestrogéniques, androgéniques ou progestiniques, cortisone et analogues corticostéroïdiens, hormone endogène, médicament auxinique, amphétamine, stimulateur de la croissance, pour la préparation d'un médicament destiné à la détoxication d'un animal traité par un médicament par la voie intramusculaire.

2. Utilisation selon la revendication 1, où l'antibiotique est choisi parmi les tétracyclines, les antibiotiques aminoglycosidiques, les céphalosporines et les pénicillines ; le médicament sympathicomimétique est un agoniste β ayant une structure de 2-phényléthylamine ; l'agent antithyroïdien est un dérivé du 2-thiouracile ; le sulfamide est substitué au niveau de l'azote du sulfamide avec un cycle isoxazole ; le stimulant de la croissance a une structure de quinoléine.

3. Utilisation selon la revendication 1, où l'antibiotique est choisi parmi l'oxytétracycline, la doxycycline, la néomycine B, la gentamicine C, la tobramycine, la kanamycine, l'amikacine et la streptomycine, le ceftazidime, le céfotaxime, la céfalexine, une pénicilline ; le sulfamide est le sulfaméthoxazole ou la sulfaméthoxazine ; le médicament antiviral est l'idoxuridine ; le médicament antimycotique est l'amphotéricine et le kétoconazole ; le médicament antituberculeux est la cyclosérine ou l'isoniazide ; le médicament sympathicomimétique est le clenbutérol ou le cimatérol ; l'agent antithyroïdien est le méthylthiouracile ou le propylthiouracile ; la substance oestrogénique est le diéthylstilbestrol, le 17-β-estradiol ou le zéranolol ; la substance androgénique est le trenbolone ou la 19-nortestostérone ; la substance progestinique est la progestérone ; la substance analogue corticostéroïdien est la dexaméthasone ; l'hormone endogène est la somatotrophine ; le médicament auxinique est choisi parmi la virginiamycine, la zincobacitracine, l'avoparcine ; le médicament amphétaminique est l'amphétamine ou la méthamphétamine, le stimulateur de la croissance est l'olaquindox.

4. Utilisation selon les revendications 1 à 3, où le rapport molaire F(ab')2 / médicament est compris entre 1/1 et 1/5.

5. Utilisation selon les revendications 1 à 3, où ladite détoxication est réalisée avant l'abattage ou la traite du lait.
